(19) Europäisches Patentamt / European Patent Office / Office européen des brevets

(11) **EP 2 932 899 A1**

(12) **EUROPEAN PATENT APPLICATION**
published in accordance with Art. 153(4) EPC

(43) Date of publication:
21.10.2015 Bulletin 2015/43

(51) Int Cl.:
*A61B 5/16* (2006.01)

(21) Application number: 13861653.7

(22) Date of filing: 13.12.2013

(86) International application number:
PCT/JP2013/007352

(87) International publication number:
WO 2014/091766 (19.06.2014 Gazette 2014/25)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME

(30) Priority: 15.12.2012 JP 2012274147

(71) Applicant: **Tokyo Institute of Technology**
**Tokyo 152-8550 (JP)**

(72) Inventor: **MIYAKE, Yoshihiro**
**Tokyo 152-8550 (JP)**

(74) Representative: **Schröer, Gernot H.**
**Meissner, Bolte & Partner GbR**
**Bankgasse 3**
**90402 Nürnberg (DE)**

(54) **APPARATUS FOR EVALUATING HUMAN MENTAL STATE**

(57)    An evaluation apparatus 100 evaluates the relationship between multiple subjects 2 in communication between the subjects 2. A non-verbal information measurement unit 10 measures non-verbal information S0a and S0b respectively obtained for the multiple subjects 2a and 2b, and quantifies the non-verbal information S0a and S0b so as to generate first signals S1a and S1b each configured as a time-series signal. A waveform analyzing unit 22 generates second signals S2a and S2b for the respective subjects, each configured as a value that relates to a feature configured as non-verbal information rhythm with respect to the corresponding one of the subjects 2a and 2b. A relationship evaluation unit 24 generates a third signal S3 configured as an index that represents a mental state with respect to the relationship between the multiple subjects, based on a relative relationship between the multiple second signals S2a and S2b that correspond to the respective subjects.

FIG.3

EP 2 932 899 A1

**Description**

[TECHNICAL FIELD]

The present invention relates to a technique for evaluating the mental state of a human being.

[BACKGROUND ART]

There is a demand for a technique for evaluating the mental state, cognitive state, or the like, of a human being based on a predetermined index in an objective manner or in a quantitative manner. Known conventional techniques for such an evaluation (which will be referred to as "evaluation of mental state") include (i) evaluations based on questionnaire results, and (ii) evaluations based on measurement results obtained by measuring physiological response of a subject, such as brain waves, heart rate, perspiration, respiration, temperature, etc. In addition, (iii) a method has been proposed in which physiological information obtained based on brain waves, heart rate, etc., or observation information obtained based on non-verbal information (sighing, nodding, tone of voice, etc.), and subjective evaluation data such as questionnaire results are acquired, the information and data thus acquired are studied, and the mental state is predicted based on the observation information (see Patent document 1).

[Related Art Documents]

[Patent Documents]

[Patent Document 1]
Japanese Patent Application Laid Open No. 2010-22649
[Patent Document 2]
Japanese Patent Application Laid Open No. 2007-97668
[Patent Document 3]
Japanese Patent Application Laid Open No. 2013-52049

[DISCLOSURE OF THE INVENTION]

[PROBLEM TO BE SOLVED BY THE INVENTION]

However, problems remain with such a questionnaire method from the quantitative viewpoint and from the real-time viewpoint. On the other hand, a physiological response measurement method requires the subject to wear sensors, thus requiring troublesome measurement procedures.
With the techniques described in Patent documents 1 and 2, the mental state of a given subject is evaluated based on the state of the subject alone (including physiological information, measurement information, etc.). However, the "interpersonal relationship" has a great effect on the mental state of an individual human being in his/her communication. This means that it is important to evaluate this "relationship". However, with conventional techniques, such evaluation is not performed based on information including the "relationship" in communication. The term "relationship" as used here is regarded as a mental state involved in the relationship between multiple individual human beings (subjects), which corresponds to the sensations or information with respect to the context-sharing which is the basis of communication. Specific examples of such a "relationship" include empathy, sense of trust, sense of identification, sense of belonging, sense of reality, consensus or agreement, sense of understanding, and the like. Such a relationship can be distinguished from individual psychological states such as likes and dislikes with respect to the other person, interest, recognition, disapprobation (difference of opinion), compromise, incomprehension (half-listening), doubt (suspicion), and the like. The "sense of belonging" represents the sense of fitting into the situation. The sense of reality represents the sense of joining in the situation.
Patent document 3 discloses a synchrony detection apparatus that detects synchrony in a conversation. The synchrony detection apparatus measures a physiological index for each of a first speaker and a second speaker for a predetermined period of time, and convolutes the measurement results so as to detect synchrony. Such a synchrony detection apparatus is designed assuming that the synchrony level becomes higher as the difference in the physiological index between the two speakers becomes smaller on the time axis, i.e., that the synchrony level becomes lower as the difference becomes larger.
The inventor has investigated the technique described in Patent document 3, and has come to recognize the following problems.

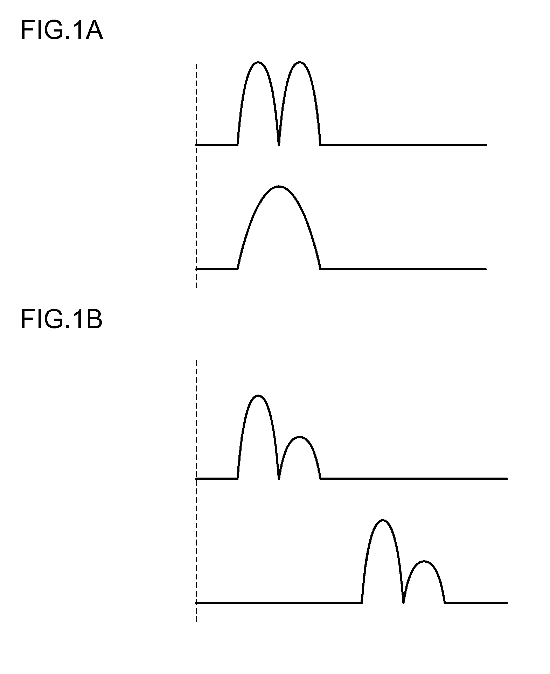

Figs. 1A and 1B are waveform diagrams each showing raw data of the physiological indexes obtained from the two subjects. Here, "S1a" and "S1b" schematically represent the raw data obtained as primary data from the first speaker and the raw data obtained as primary data from the second speaker, respectively. With the technique described in Patent document 3, judgment is made that a case shown in Fig. 1A exhibits a higher synchrony level than that in a case in Fig. 1B.

However, as an investigation result obtained by the present inventor, directing attention to the mental states of multiple speakers (subjects), it has been found that the synchrony level is not always high when there is no difference in the physiological index on the time axis as shown in Fig. 1A. That is to say, in some cases, the synchrony level is high when the waveforms have a similar shape although there is a large difference in the physiological index on the time axis. As described above, with the evaluation method described in Patent document 3, in some cases, such an arrangement cannot provide an index that reflects the mental state between multiple subjects.

The present invention has been made in view of such a situation. Accordingly, it is an exemplary purpose of an embodiment of the present invention to provide a technique for evaluating the relationship between multiple individual human beings in a real-time manner using an approach that differs from those of conventional techniques.

[MEANS TO SOLVE THE PROBLEM]

An embodiment of the present invention relates to an evaluation apparatus that evaluates a relationship between multiple subjects in a communication between the subjects. The evaluation apparatus comprises: a non-verbal information measurement unit that observes each of the multiple subjects, and that generates first signals each of which is obtained as a time-series signal by quantifying non-verbal information obtained from the corresponding subject; a waveform analyzing unit that generates, based on the first signals respectively obtained for the multiple subjects, second signals each of which is configured as a value that relates to a feature configured as a rhythm of the non-verbal information with respect to the corresponding subject; and a relationship evaluation unit that generates a third signal configured as an index that represents a mental state with respect to the relationship between the multiple subjects, based on a relative relationship between the multiple second signals that respectively correspond to the multiple subjects.

The present inventor has found that there is variation in a relative relationship between time-series signals of non-verbal information obtained based on the activity of each subject, and particularly, a relative relationship between values that relate to a feature configured as a rhythm of non-verbal information (which will be referred to as the "rhythm relationship value"), according to a mental state with respect to the relationship between the subjects. Such an embodiment is capable of evaluating the mental state with respect to the relationship between the subjects, based on the third signal that corresponds to the relative relationship between the multiple rhythm relationship values of the non-verbal information.

It should be noted that any combination of the aforementioned components may be made, and any component of the present invention or any manifestation thereof may be mutually substituted between a method, apparatus, and so forth, which are effective as an embodiment of the present invention.

[ADVANTAGE OF THE PRESENT INVENTION]

An embodiment of the present invention is capable of evaluating the relationship between multiple subjects.

[BRIEF DESCRIPTION OF THE DRAWINGS]

| | |
|---|---|
| Figs. 1A and 1B | are waveform diagrams each showing an example of raw data that represents a physiological index obtained from the corresponding one of two subjects; |
| Fig. 2 | is a diagram showing an evaluation apparatus according to an embodiment; |
| Fig. 3 | is a diagram showing a specific configuration of the evaluation apparatus; |
| Figs. 4A and 4B | are waveform diagrams respectively showing a first signal and a second signal acquired in an experiment described in an embodiment 1; |
| Fig. 5A | is a waveform diagram showing the second signal acquired in the experiment described in the embodiment 1, and Figs. 5B and 5C are correlation diagrams each showing a correlation between the second signals in a different time slot; |
| Figs. 6A and 6B | are diagrams for describing the second signal according to an embodiment 2; |
| Fig. 7A | is a waveform diagram showing the second signal acquired in an experiment described in the embodiment 2, and Fig. 7B is a waveform diagram showing a third signal; |

Fig. 8        is a waveform diagram showing a relationship between the third signal and the mental state according to the embodiment 2;

Fig. 9A        is a waveform diagram showing the first signal according to the embodiment 2, Fig. 9B is a waveform diagram showing the second signals, Fig. 9C is a correlation diagram showing a correlation between the second signals, and Fig. 9D is a correlation diagram showing a correlation between the first signals;

Fig. 10        is a waveform diagram showing the first signal and the second signal according to an embodiment 3;

Fig. 11        shows a histogram of the third signal acquired in an experiment described in the embodiment 3;

Fig. 12        is a waveform diagram showing the first signal and the second signal acquired in an experiment described in an embodiment 4;

Fig. 13        is a diagram for describing the third signal according to the embodiment 4;

Fig. 14A        is a waveform diagram showing the second signal according to an embodiment 5, and Fig. 14B shows a histogram of a synchronization rate; and

Fig. 15        is a diagram showing the evaluation apparatus according to the embodiment 5.

[BEST MODE FOR CARRYING OUT THE INVENTION]

Description will be made below regarding preferred embodiments according to the present invention with reference to the drawings. The same or similar components, members, and processes are denoted by the same reference numerals, and redundant description thereof will be omitted as appropriate. The embodiments have been described for exemplary purposes only, and are by no means intended to restrict the present invention. Also, it is not necessarily essential for the present invention that all the features or a combination thereof be provided as described in the embodiments.

As a result of investigation conducted by the present inventor, mental activity such as the interests, emotions, etc. of a human being, i.e., the mental state, is reflected in the movement of a human being, i.e., the non-verbal information dynamics. Familiar examples of such dynamics include a rhythm such as back channeling or nodding in responses that accompany communication. With the present invention, the rhythm of such non-verbal information dynamics is analyzed so as to evaluate the mental state directing attention to the non-verbal information dynamics.

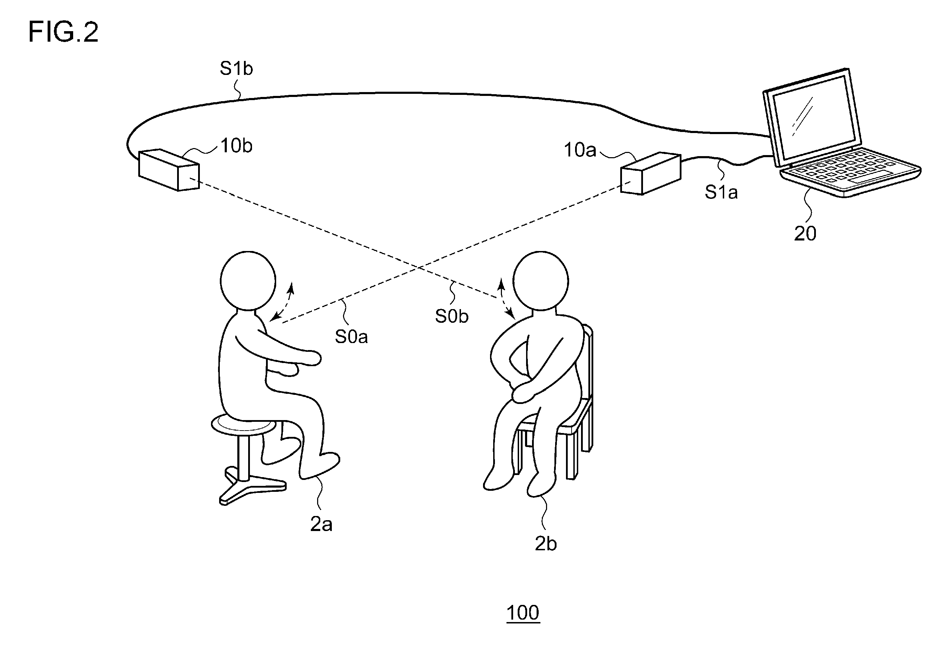

Fig. 2 is a diagram showing an evaluation apparatus 100 according to an embodiment. The evaluation apparatus 100 evaluates the mental states of multiple human beings (subjects) 2a and 2b in communication between them. The mental state as used here can be classified into two aspects, i.e., an individual aspect and an interpersonal relationship aspect. It is a main purpose of the evaluation apparatus 100 to evaluate the latter aspect, i.e., the interpersonal relationship aspect. The evaluation of the mental state as used here is not restricted to evaluation of conscious action. Also, the mental state as used here may include subconscious states of a human being such as emotions, empathy, sense of identification, and the like.

Furthermore, the present inventor has found that the individual aspect (which will also be referred to in the present specification as the "mental state individual aspect"), which is one of the aspects of the mental state, shows a marked tendency to be reflected in the amplitude, frequency, or the like, of the non-verbal information dynamics obtained from each subject. In contrast, the interpersonal relationship aspect (which will also be referred to as the "mental state relationship aspect"), which is the other one of the aspects of the mental state, shows a marked tendency to be reflected in the relative relationship between multiple items of non-verbal information obtained with respect to the multiple subjects 2, and particularly, the relationship between values each regarded as rhythms. The evaluation apparatus 100 according to the embodiment evaluates the mental state of the multiple subjects 2 based on the findings described above.

Specifically, the evaluation apparatus 100 evaluates the relationship between subjects in interpersonal communication between the multiple subjects 2a and 2b. Examples of the relationship between the subjects, i.e., the mental states with respect to the relationship between the subjects, as used here, include empathy, sense of trust, sense of identification, sense of belonging, sense of reality, consensus or agreement, sense of understanding, and the like. The evaluation apparatus 100 evaluates at least one from among the mental states with respect to the relationship between the subjects, or a desired combination of these. The mental state with respect to the relationship between the subjects can be distinguished from the emotional responses of each subject toward the other subject.

For simplicity of description and ease of understanding, description will be made in the present embodiment regarding an example in which the evaluation apparatus 100 evaluates the relationship between two subjects.

For example, the subjects 2a and 2b face each other and communicate with each other in the same space. The kind of communication is not restricted in particular. Rather, various kinds of communication may be employed. Examples of such communication include everyday conversations, discussions, presentations, lectures, and the like.

Such communication does not require the subjects 2a and 2b to be in the same space. Also, the evaluation apparatus 100 is applicable to communication made via a telephone, teleconference system, or the like.

The evaluation apparatus 100 according to the embodiment monitors non-verbal information that can be externally measured as visual data, audio data, or the like, instead of physiological information with respect to the subjects 2a and 2b. Examples of such measurable non-verbal information include nodding, body language, gestures, trunk movement, gaze retention time, tone of voice, sighing, non-verbal information with respect to turn-taking (speaking length, pose length, speaking rate, speaking timing, etc.), and non-verbal information with respect to speaking such as voice pitch, intonation, and the like.

A non-verbal information measurement unit 10 includes a camera or a microphone, a sensor (acceleration sensor, velocity sensor, gyroscope) for measuring the movement, a sensor for measuring spatial position, and other sensors. The non-verbal information measurement unit 10 measures non-verbal information S0a and S0b obtained from the subjects 2a and 2b, and generates a time-series signal (which will be referred to as a "first signal S1" hereafter) obtained by quantifying the non-verbal information. The kind of non-verbal information measurement unit 10 may preferably be selected according to the non-verbal information S0 to be measured. It should be noted that the first signal S1 corresponds to the physiological index as described in

Patent document 3.

A signal processing unit 20 generates, based on the multiple first signals S1a and S1b, a third signal S3 configured as a mental state index between the multiple subjects 2. Furthermore, the signal processing unit 20 generates fourth signals S4a and S4b that respectively represent the mental states of the multiple subjects 2 based on the first signals S1. The above is the schematic description of the evaluation apparatus 100.

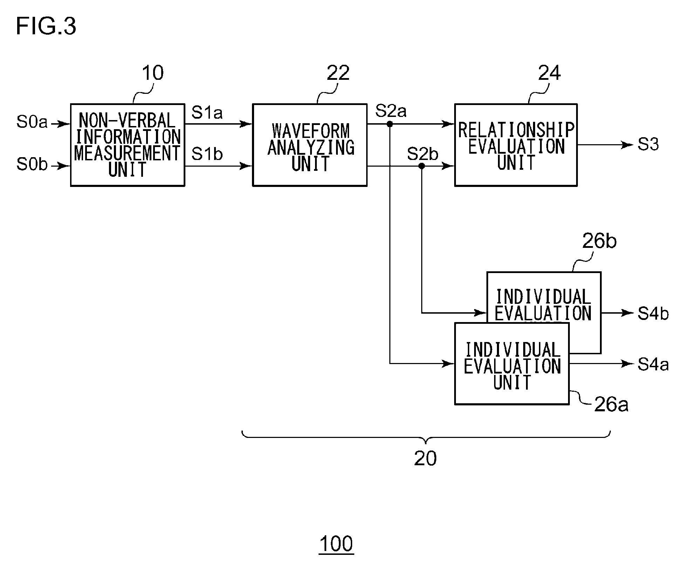

Fig. 3 is a diagram showing a specific configuration of the evaluation apparatus 100.

The evaluation apparatus 100 includes a non-verbal information measurement unit 10, a waveform analyzing unit 22, a relationship evaluation unit 24, and an individual evaluation unit 26.

As described above, the non-verbal information measurement unit 10 measures the non-verbal information that can be obtained from the multiple subjects 2a and 2b. Furthermore, the non-verbal information measurement unit 10 respectively generates the first signals S1a and S1b each configured as a time-series signal obtained by quantifying the non-verbal information.

The signal processing unit 20 shown in Fig. 2 includes the waveform analyzing unit 22, the relationship evaluation unit 24, and the individual evaluation unit 26. In many cases, the first signal S1 obtained by quantifying the actions of the subject cannot appropriately be used as it is to evaluate the mental state of the subject 2. In order to solve such a problem, the waveform analyzing unit 22 generates a second signal S2 based on the first signal S1. The second signal S2 is a time-series signal (rhythm relationship value) that relates to the rhythm characteristics of the non-verbal information.

Examples of the second signal S2 configured as a rhythm relationship value of the non-verbal information will be illustrated below.

(i) A time-series signal obtained by performing statistical processing or signal processing on the first signal S1 for each period.
For example, the second signal may be generated by calculating the average, variance, or standard deviation of the first signal, or otherwise by filtering the first signal.
(ii) A time-series signal obtained by time differentiating or otherwise time integrating the first signal S1.
For example, in a case in which the first signal has a velocity dimension, the second signal S2 has an acceleration dimension or otherwise has a displacement dimension.
(iii) A time series signal obtained by coarse-graining the first signal S1.
Examples of such a second signal S2 include an envelope curve of the first signal S1, and the like.
(iv) A time-series signal with respect to information that represents the waveform of the first signal S1.
Specific examples of such a time-series signal include (iv-1) waveform kind, (iv-2) spectrum, (iv-3) frequency, (iv-4) duty ratio, (iv-5) amplitude, (iv-6) extracted data of geometric features of the waveform pattern, and the like.

For example, a rhythm pattern "1, 2, 3" is recognized as a pattern that differs from a rhythm pattern "1-2-3". The second signal S2 is selected from among the signals listed above as examples in (i) through (iv), such that it represents the difference between such rhythm patterns. It has been found by the present inventor that such a second signal S2 is preferably configured as one from among (a) time-series data of the frequency information with respect to the first signal S1, (b) time-series data of the phase information with respect to the first signal S1, and (c) a combination of (a) and (b).

Preferable examples of (a) include: (a-1) time-series data of the magnitude (amplitude or otherwise power spectrum) of the frequency component of a predetermined frequency; (a-2) time-series data of the frequency component that exhibits the maximum magnitude; and the like.

Preferable examples of (b) include: (b-1) time-series data of the phase of a predetermined frequency (or frequency band); (b-2) time-series data of the phase of occurrence of a predetermined event that can be detected based on the first signal S1; and the like.

In addition to the aforementioned example signals as listed above, other kinds of signals can be used as the rhythm relationship value, which can be clearly understood by those skilled in this art, and which are encompassed in the technical scope of the present invention. Also, the generating method (signal processing method) for generating the second signal S2 may preferably be selected according to the kind of second signal S2. That is to say, the generating method for generating the second signal S2 is not restricted in particular.

The relationship evaluation unit 24 generates the third signal S3, which is an index that represents the mental state between the multiple subjects 2a and 2b, based on the relative relationship between the multiple signals S2a and S2b that respectively correspond to the multiple subjects 2a and 2b. Here, examples of such a relative relationship between the multiple second signals S2a and S2b include: (i) degree of synchrony, (ii) phase difference, (iii) correlation, (iv) frequency relationship, (v) phase relationship, (vi) amplitude relationship, (vii) relationship between the geometric features each configured as a waveform pattern, and a desired combination of these. Specifically, as described later, it has been confirmed beforehand by inspection that, in the communication between the multiple subjects, when they agree with each other, the phase difference in the rhythm relationship value between the subjects becomes stable, the number of oscillations (frequencies) of the signals approach each other, or otherwise the strength of the correlation between them becomes higher.

For example, the correspondence between the relative relationship between the multiple second signals S2a and S2b and the mental state between the multiple subjects may be studied by experiment or inspection, and may be stored in a database. Also, a correspondence newly obtained in actual operation of the evaluation apparatus 100 may be studied and may be stored in the database.

The third signal S3 is configured as an index of the mental state between the individuals, examples of which include empathy, sense of trust, sense of identification, sense of belonging, sense of reality, consensus or agreement, sense of understanding, and the like. The third signal S3 is acquired as 1/0 binary data, multivalued data, or otherwise vector data.

The kind of first signal configured as the non-verbal information, the kind of second signal configured as a rhythm relationship value obtained based on the first signal, and the kind of relative relationship between the multiple second signals are selected and determined according to the kind of mental state between the multiple subjects 2a and 2b to be evaluated as the final evaluation value. Also, the kind of first signal, the kind of second signal, and the kind of the relative relationship between the second signals are determined giving consideration to results obtained beforehand by experiment or inspection.

The individual evaluation unit 26 generates the fourth signals S4a and S4b, which are indexes that respectively represent the mental states of the multiple subjects 2a and 2b, based on the second signals S2a' and S2b' respectively obtained for the multiple subjects 2a and 2b. The second signals S2a' and S2b', which are to be input to the individual evaluation unit 26, may be the same as the second signals S2a and S2b input to the relationship evaluation apparatus 24. Also, the second signals S2a' and S2b' may be configured as different signals obtained by performing signal processing on the second signals S2a and S2b by means of the waveform analyzing unit 22.

The above is the configuration of the evaluation apparatus 100.

Description will be made below regarding the evaluation of the level of agreement between the multiple subjects, which is configured as a mental state between them.

As an investigation result uniquely recognized by the present inventor, it has been found that, as the non-verbal information S0 that can be measured by the non-verbal information measurement unit 10, nodding actions of the subject 2 can be effectively used to evaluate the synchrony level.

The non-verbal information measurement unit 10 measures the nodding actions of the multiple subjects 2a and 2b, i.e., their chin movement, and quantifies the measurement results so as to generate the first signals S1a and S1b. The non-verbal information measurement unit 10 may be configured by combining a camera and an image processing apparatus. Such a camera may be provided for each subject. Also, a single camera may be employed to measure all the nodding actions of the multiple subjects. Also, if the situation permits it, a velocity sensor or an acceleration sensor may be attached to each of the subjects 2a and 2b so as to measure the nodding actions S0a and S0b.

It should be noted that, in a case in which evaluation is made giving consideration to the spatial relationship between the multiple subjects 2a and 2b, a position sensor may be provided as described later.

The waveform analyzing unit 22 receives the first signals S1a and S1b respectively obtained for the multiple subjects 2a and 2b, and performs predetermined signal processing on the first signals S1a and S1b thus received. Description will be made below regarding specific examples of signal processing with reference to the embodiments.

[Embodiment 1]

The first signals S1a and S1b are obtained by quantifying the actual measurement results obtained by measuring chin movement of the subjects 2 in a face-to-face conversation. In the embodiment 1, the waveform analyzing unit 22 calculates the time average of the amplitude of the first signal S1 so as to generate the second signal S2.

In order to confirm the appropriateness of the embodiment 1, an experiment was performed. In this experiment, one of the subjects performed the role of a teacher, and the other performed the role of a student. The subject 2a who performed the role of a teacher provided an explanation with respect to a predetermined theme, and the subject 2b who performed the role of a student understood the explanation. Only the subject 2a who performed the role of a teacher was allowed to speak. This experiment was performed for twelve male students and eight female students in their twenties, and specifically, for ten pairs each comprising two individuals from among them.

In this experiment, a three-dimensional acceleration sensor was attached to each of the subjects 2a and 2b. More specifically, with the vertical direction as the X-axis direction, and with the gaze direction as the Z-axis direction, the acceleration $x(t)$ in the X-axis direction and the acceleration $z(t)$ in the Z-axis direction were measured, and the norm of these values, which is represented by $\sqrt{(x^2(t) + z^2(t))}$, was employed as the first signal S1.

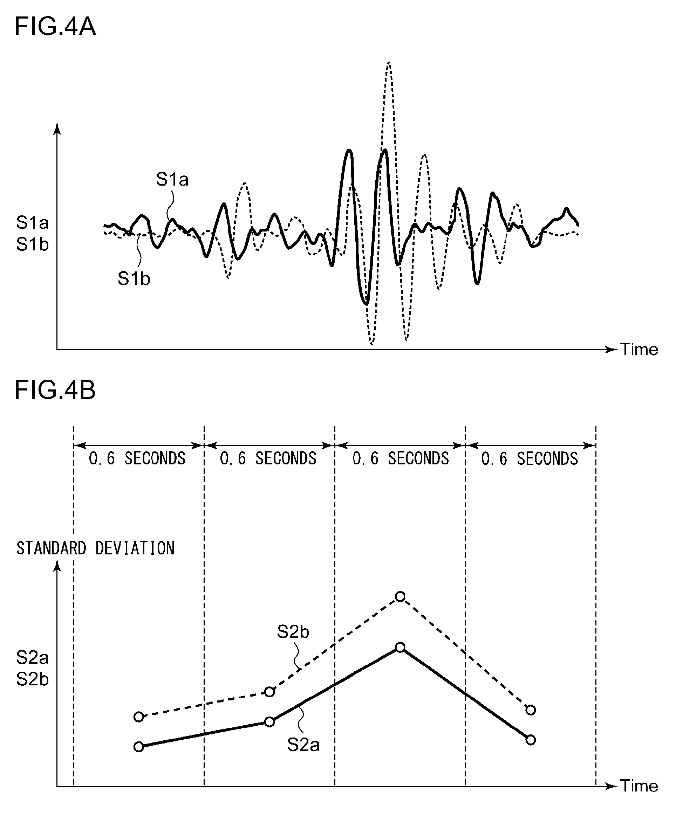

Fig. 4 is a waveform diagram showing the first signal S1 obtained by the experiment 1 and the second signal S2 obtained based on the first signal S1 according to the embodiment 1.

In the embodiment 1, the second signals S2a and S2b are generated by calculating, every 0.6 seconds, the standard deviation (SD) of the first signals S1a and S1b obtained by means of the non-verbal information measurement unit 10. The second signals S2 and S2b thus generated each correspond to the amplitude of the nodding action. There is great variation in the acceleration in a period of time in which nodding action occurs. Thus, the second signal S2 is configured as a rhythm relationship value that represents the nodding action.

After this experiment, it has been confirmed by inspection that, directing attention to the second signals S2a and S2b respectively obtained for the two subjects 2a and 2b, in the period T1 in which the level of agreement is low, synchrony does not occur between the two second signals S2a and S2b, i.e., synchrony does not occur between the amplitudes of the nodding actions. In contrast, in the latter period T2 in which the level of agreement is high, synchrony occurs between the two second signals S2a and S2b, i.e., synchrony occurs between the amplitudes of the nodding actions, as compared with that in the period T1. That is to say, this suggests that the time waveforms of the amplitudes of the nodding actions of the multiple subjects have the potential to change from an asynchronous state to a synchronous state when the subjects agree with each other.

The relationship evaluation unit 24 generates the third signal S3, which is an index that represents the relationship between the subjects 2a and 2b, based on the relative relationship between the second signals S2a and S2b respectively obtained for the two subjects 2a and 2b, and specifically, based on the presence or absence of synchrony.

The level of synchrony or synchronization between two time-series signals may preferably be evaluated using known techniques. That is to say, with the present invention, such an evaluation method is not restricted in particular. For example, the degree of correlation (correlation coefficient r) between the two waveforms may be calculated so as to evaluate the synchrony level or synchronization level between the two signals. Also, the waveform difference between the two signals may be calculated as a simple index, and the synchrony level or synchronization level between the two signals may be evaluated based on the waveform difference thus calculated.

With the present embodiment, the relationship evaluation unit 24 calculates the correlation coefficient r between the second signals S2a and S2b for each time slot TS.

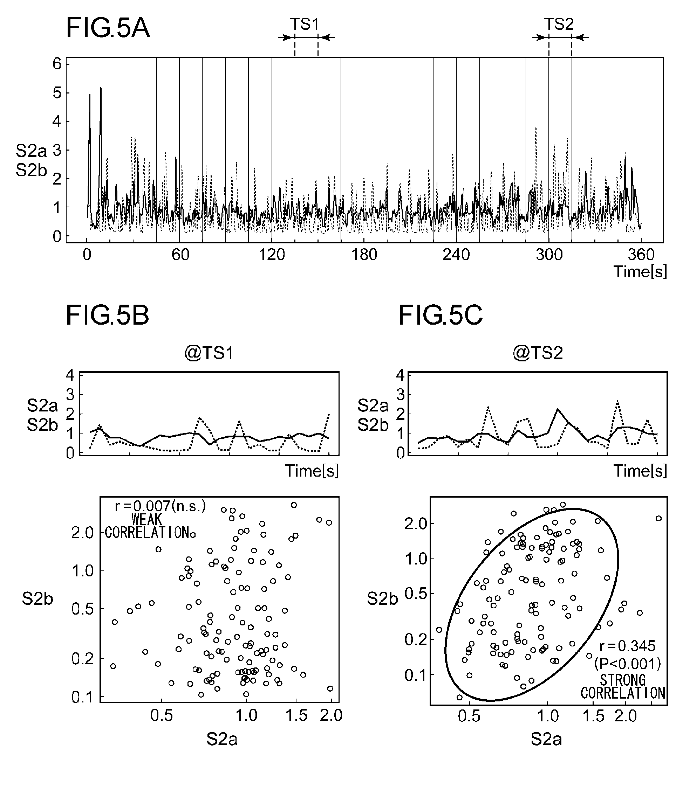

Fig. 5A is a waveform diagram showing the second signal S2 acquired in the experiment described in the embodiment 1. Figs. 5B and 5C are correlation diagrams each showing a correlation between the second signals S2 obtained for different time slots. Here, each time slot has a length of 15 seconds.

In the time slot TS1, the correlation coefficient r has a small value of 0.007. In contrast, the correlation coefficient r in the time slot TS2 has a value of 0.345 ($p < 0.001$), which indicates a strong correlation.

The p value, which is calculated as an index of statistical significance, has a value of $p < 0.001$ for the time slot TS2, which means that there is a high level of statistical significance.

After the experiment, verification interviews were performed for the subjects 2a and 2b. The state of understanding of the subject who performed the role of a student was evaluated so as to evaluate the synchrony level for each time slot. As a result, it has been found that there is a strong correlation between the second signals S2a and S2b (i.e., the amplitudes of nodding actions) in a time slot in which the second signals S2a and S2b each have a large value. Furthermore, it has been found that, in many cases, there is empathetic communication in a time slot in which the correlation coefficient r has a large value. That is to say, it has been confirmed that the correlation coefficient r obtained in this embodiment functions as an index that represents the interpersonal mental state between the subjects. Thus, the relationship evaluation unit 24 may output the correlation coefficient r as the third signal S3. Also, the relationship evaluation unit 24 may output the correlation coefficient r in the form of discrete-valued data.

In summary, the time waveform of the amplitude of the nodding action may be used as the second signal S2. Also, the degree of synchrony between the second signals S2 respectively obtained for the multiple subjects 2 may be evaluated as an index that represents the mental state relationship between the multiple subjects 2, thereby evaluating the synchrony level.

[Embodiment 2]

In this embodiment, the second signal S2, which is a rhythm relationship value, is generated directing attention to the frequency component of the first signal S1. The first signal S1 is configured as a signal that represents a nodding action in the same way as in the embodiment 1. More specifically, the first signal S1 is configured as a norm of the acceleration in the X direction and the acceleration in the Z direction. The waveform analyzing unit 22 converts the first signal S1 into frequency-domain data. Such conversion may be performed using a fast Fourier transform method or the like.

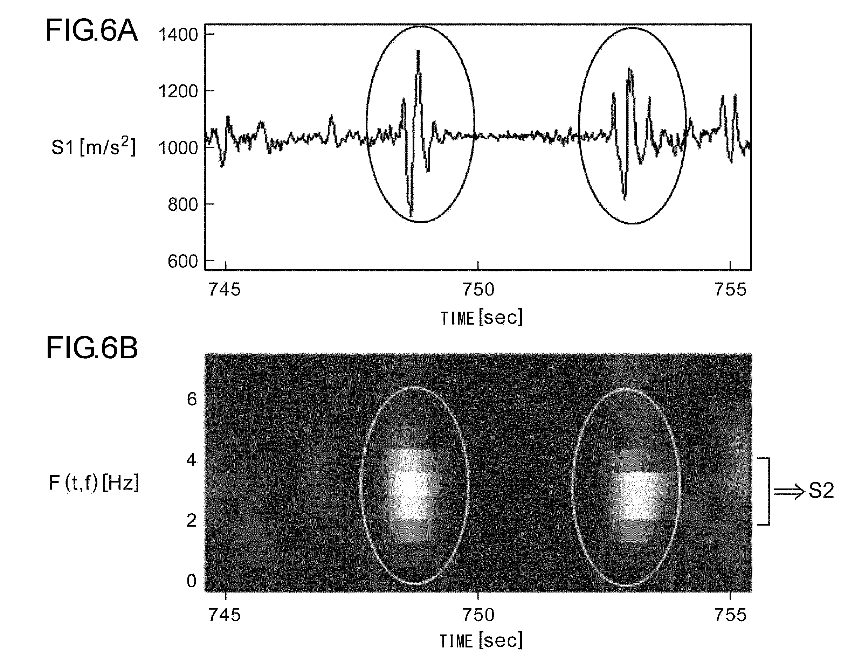

Figs. 6A and 6B are diagrams for describing the second signal S2 according to the embodiment 2. Fig. 6A shows the first signal S1. Fig. 6B shows the frequency-domain data F(t,f) obtained as a Fourier transform of the first signal S1. In Fig. 6B, the horizontal axis represents the time axis, the vertical axis represents the frequency axis, and the shading represents the magnitude (power spectrum) of the frequency-domain data F(t,f). The nodding action has a dominant frequency component ranging between 2 and 4 Hz. Thus, the energy (power) may be integrated in the frequency direction over the frequency domain ranging between 2 and 4 Hz, so as to generate the second signal S2 configured as a rhythm relationship value.

$$S2(t) = \int F(t, f) \, df.$$

In order to confirm the appropriateness of the embodiment 2, the following experiment was performed. In this experiment, two subjects 2 cooperated with each other in order to resolve a problem. The point of difference between the first embodiment and the second embodiment is that, in the second embodiment, both the two subjects were allowed to have a conversation with each other, i.e., bi-directional communication was performed. As such a problem to be resolved, the two subjects cooperated with each other to estimate the rent of an apartment based on the layout of the apartment and other information. This experiment was performed for six male students and four female students in their twenties, and specifically, for five pairs each comprising two individuals from among them.

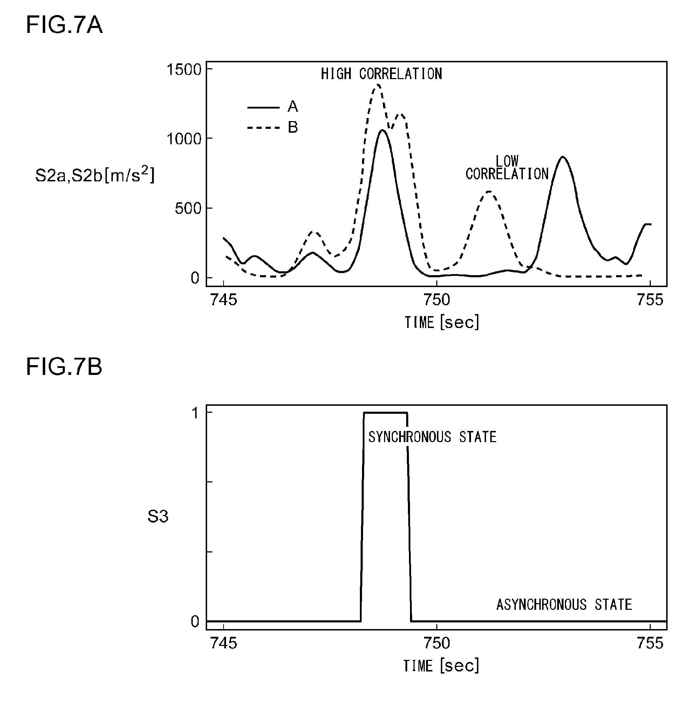

Fig. 7A is a waveform diagram showing the second signals S2a and S2b obtained by experiment according to the embodiment 2. Fig. 7B is a waveform diagram showing the third signal S3. The correlation coefficient r between the second signals S2a and S2b is calculated for every predetermined time period. The correlation coefficient r thus calculated is converted into binary data that is set to a synchronous state (1) or an asynchronous state (0) by means of threshold judgment, so as to generate the third signal S3.

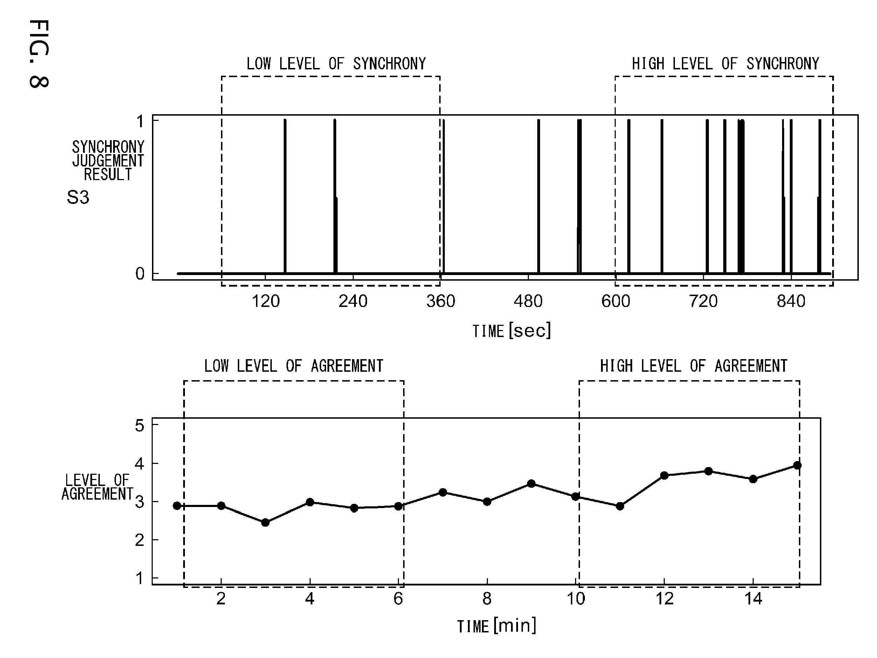

Fig. 8 is a waveform diagram showing the relationship between the third signal S3 and the mental state. The mental state was obtained by a questionnaire after the experiment, and is represented by the average of the levels of agreement between the two subjects evaluated on a scale of 1 to 5. In the approximately 15 minutes of discussion, the third signal S3 exhibits 1 at a low frequency during a first period of 5 minutes (ranging between minute 1 to minute 6) in which the level of agreement is relatively low. In contrast, during a final period of 5 minutes (ranging between minute 10 to minute 15), the third signal S3 exhibits 1 at a high frequency. Thus, it can be said that the third signal S3 itself, or otherwise a value obtained by time-averaging the third signal S3, represents the interpersonal mental state between the multiple subjects 2.

Description will be made below regarding the advantage of the evaluation apparatus 100 according to the embodiments as compared with conventional technique described in Patent document 3.

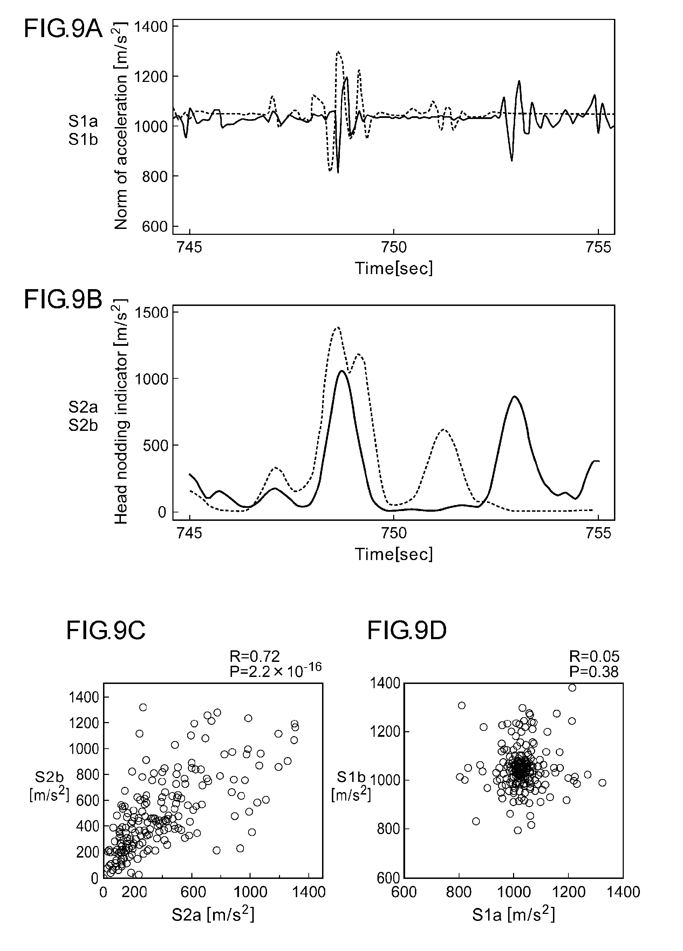

Fig. 9A is a waveform diagram showing the first signals S1a and S1b according to the embodiment 2. Fig. 9B is a waveform diagram showing the second signals S2a and S2b. Fig. 9C is a correlation diagram showing the correlation between the second signals S2a and S2b. Fig. 9D is a correlation diagram showing the correlation between the first signals S1a and S1b. In a case in which the correlation coefficient r is calculated based on the correlation shown in Fig. 9D, the correlation coefficient r is 0.05. In this case, a correlation is not detected between the two signals. That is to say, with such a conventional technique, evaluation is performed directing attention to only the correlation between the first signals S1a and S1b each obtained as primary raw data for each subject. Thus, in some cases, synchrony or agreement cannot be detected even if there is synchrony or agreement between the subjects. In contrast, with the evaluation apparatus 100 according to the embodiment, the first signals S1a and S1b are converted into the second signals S2a and S2b each configured as a rhythm relationship value, and the correlation between the second signals S2a and S2b thus converted is evaluated. Thus, such an arrangement is capable of detecting such synchrony, agreement, or the like.

[Embodiment 3]

In this embodiment, the second signal S2 configured as a rhythm relationship value is generated directing attention to the phase component of the first signal S1. The first signal S1 is configured as a signal that represents a nodding action in the same way as in the embodiments 1 and 2. More specifically, the first signal S1 is configured as a norm of the acceleration in the X direction and the acceleration in the Z direction.

The rhythm information is not restricted to the frequency information. The rhythm information can also be represented by the phase information. In the embodiment 3, the waveform analyzing unit 22 uses, as the second signal S2, the phase information, i.e., a phase at which a predetermined event, which can be detected based on the first signal S1, occurs. Specifically, the waveform analyzing unit 22 calculates a moving average of each of the first signals S1a and S1b over a predetermined period. Furthermore, the waveform analyzing unit 22 compares each of the moving averages thus calculated with a predetermined threshold value, so as to detect a conspicuous nodding action. After such a conspicuous nodding action is detected, the moving averages are continuously monitored so as to detect a time point (phase) at which a nodding action peak occurs. In the embodiment 3, the occurrence of the nodding action peak is detected as an event. Furthermore, the time point of the occurrence of such an event is used as the second signal S2.

In order to confirm the appropriateness of the embodiment 3, an experiment was performed. In this experiment, one of the subjects performed the role of a teacher, and the other performed the role of a student. The subject 2a who performed the role of a teacher provided an explanation with respect to a predetermined theme, and the subject 2b who performed the role of a student understood the explanation. The lecture was performed via a TV monitor. The subject who performed the role of a teacher provided an explanation for the subject who performed the role of a student in an unidirectional manner. This experiment was performed for twelve male students and eight female students in their twenties, and specifically, for ten pairs each comprising two individuals from among them.

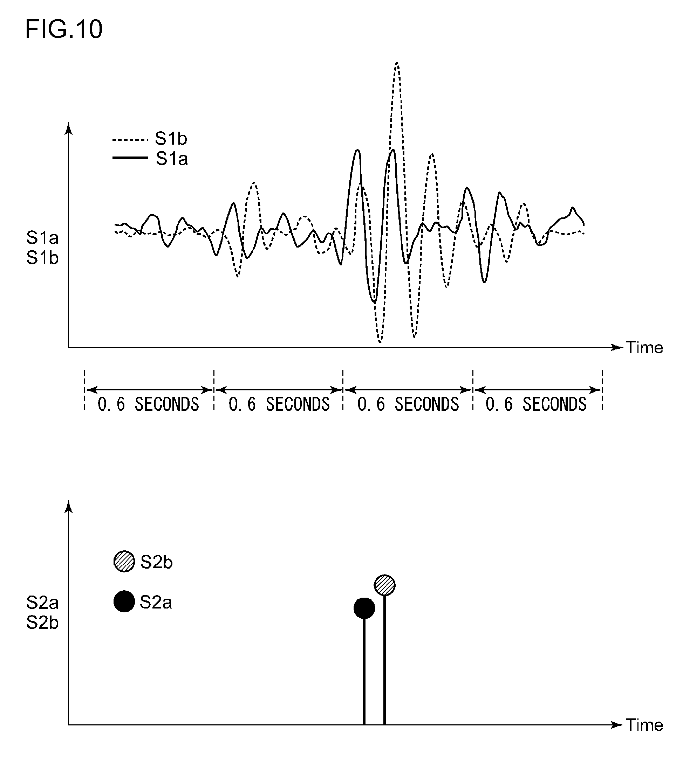

Fig. 10 is a waveform diagram showing the first signal S1 and the second signal S2 according to the embodiment 3.

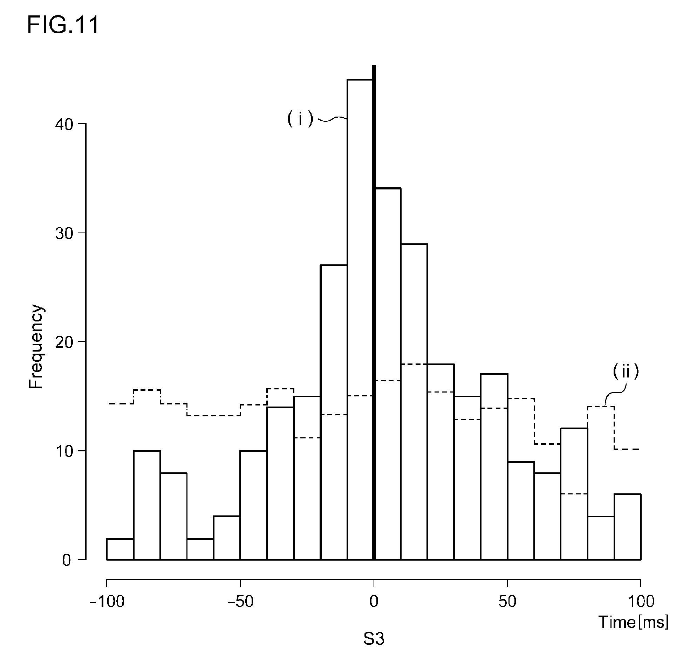

The relationship evaluation unit 24 uses, as the third signal S3, the phase difference between the second signals S2a and S2b. The third signal S3 corresponds to the phase difference in rhythm between the nodding actions. Fig. 11 shows a histogram of the third signal S3. When there is interpersonal synchrony between two subjects, there is synchronization in the nodding timings. In this case, as represented by the solid line (i) in Fig. 11, the histogram has a peak at a certain value (0 ms in this example), and has a distribution with the peak position as its center. In contrast, when synchrony does not occur, the nodding timing varies at random. In this case, as represented by the broken line (ii) in Fig. 11, the histogram has no peak and the distribution is flat.

With the embodiment 3 as described above, such an arrangement is capable of evaluating the interpersonal mental state directing attention to the phase information used as a rhythm relationship value.

[Embodiment 4]

In this embodiment, the point in common with the embodiment 2 is that the second signal S2 configured as a rhythm relationship value is generated directing attention to the frequency component of the first signal S1. Directing attention to the body movement of the subject instead of the nodding action, the first signal S1 is configured as the norm of the accelerations in the X direction, Y direction, and Z direction acquired by means of acceleration sensors attached to the subject's body.

$$S1 = \sqrt{(x^2(t) + Y^2(t) + z^2(t))}$$

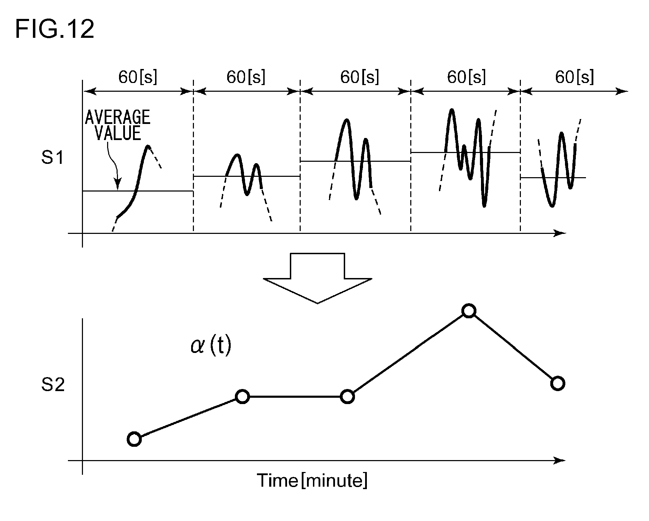

The waveform analyzing unit 22 converts the first signal S1 into frequency-domain data. For example, the waveform analyzing unit 22 may measure the number of times the first signal S1 crosses the time average of the first signal S1 itself for a predetermined period of time (e.g., 10 seconds), and may acquire, based on the measurement value, the second signal S2 that represents the number of oscillations (frequency). For example, the second signal S2 is configured as an average value obtained by averaging, over 1 minute, the frequency that is measured for every 10 seconds. Fig. 12 is a waveform diagram showing the first signal S1 and the second signal S2 according to the embodiment 4. It should be noted that, in the embodiment 4, the second signal S2 may be calculated using a fast Fourier transform method.

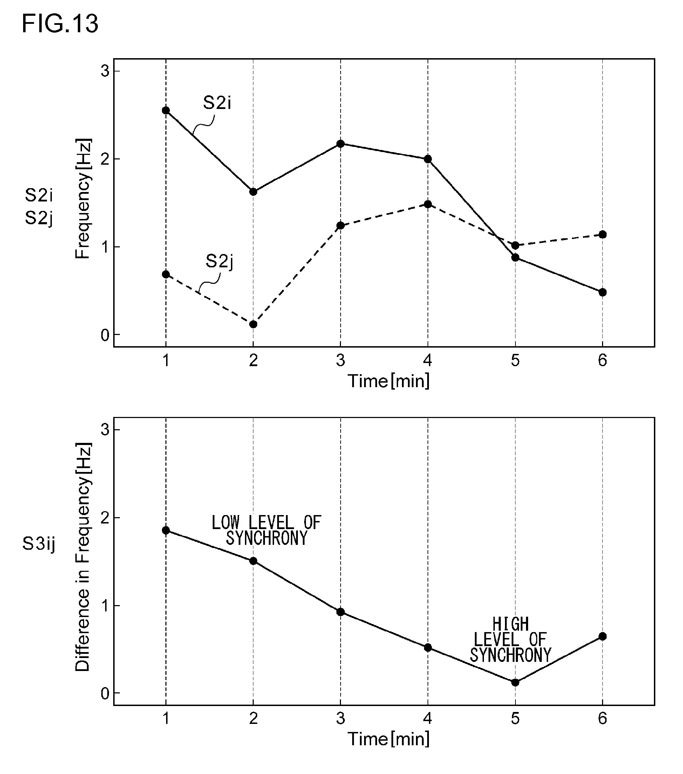

The relationship evaluation unit 24 evaluates the mental state between the multiple subjects based on the second signals S2 each corresponding to the frequency $\omega$ of the body movement of the corresponding subject. Fig. 13 is a diagram for describing the third signal S3 according to the embodiment 4. The embodiment 4 is made directing attention to the difference $A\omega_{ij}(t)$ between the frequency $\omega_i(t)$ obtained from the i-th subject and the frequency $\omega_j(t)$ obtained from the j-th subject. From the experiment results obtained by the present inventor, it has been found that the synchrony level is higher as the frequency difference $\Delta\omega_{ij}(t)$ exhibits a smaller value.

With the embodiment 4 as described above, such an arrangement is capable of evaluating the interpersonal mental state directing attention to the frequency information configured as a rhythm relationship value, and more specifically, the frequency difference between body movements.

[Embodiment 5]

In this embodiment, in the same way as in the embodiment 4, the frequency $\omega(t)$ of the body movement of the subject is used as the second signal S2 directing attention to the frequency information with respect to the body movement.
The relationship evaluation unit 24 evaluates the mental state between the subjects directing attention to the direction of change in each of the frequencies $\omega_i(t)$ and $\omega_j(t)$, instead of the difference $\Delta\omega_{ij}(t)$ between the frequencies $\omega_i(t)$ and $\omega_j(t)$.
In the present embodiment, as an index that represents the synchrony level between the i-th subject and the j-th subject, a synchronization rate $S_{ij}$ is defined as represented by the following Expression (1).

$$S_{ij} = \Sigma_{t \in Tij}\, g(\Delta x_i(t) \bullet \Delta x_j(t))/|I_{ij}| \;\ldots(1)$$

Here, $x_i(t_k)$ is the second signal obtained from the i-th subject. With the sampling period as $\Delta t$, $t_k$ is represented by $t_k = k \times \Delta t$. "$\Delta x_i(t_k)$" represents an amount of variation of $x_i(t_k)$ obtained from the i-th subject, which is represented by $x_i(t_k)$ - $x_i(t_{k+1})$. This value can also be regarded as a differential value of $x_i(t_k)$. The function g(a) is configured such that, when a > 0, it returns +1, and such that, when a < 0, it returns -1. "$T_{ij}$" represents a time period. The frequency $\omega_i(t)$, which is selected as the second signal S2, is used as the function $x_i(t)$. In this case, $\Delta x_i(t_k)$ corresponds to a variation in the frequency.
In order to confirm the appropriateness of the embodiment 5, an experiment was performed. In this experiment, a number of subjects communicated freely with a voluntarily selected companion. The synchronization rate $S_{ij}$ may be calculated for all the subject pairs.

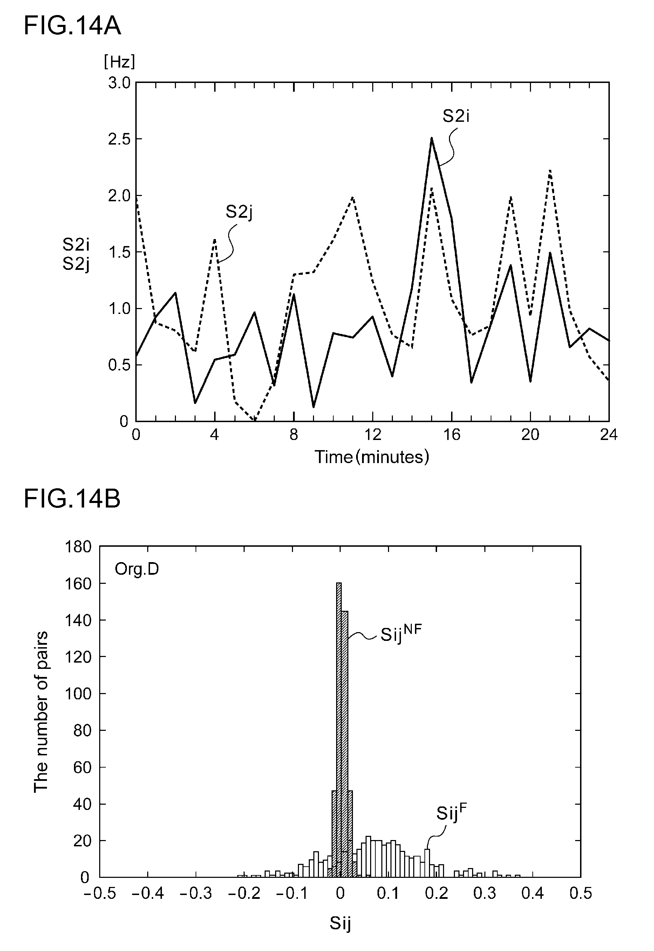

Fig. 14A is a waveform diagram showing the second signal S2 according to the embodiment 5. Fig. 14B shows a histogram of the synchronization rate $S_{ij}$. The first period $T_{ij}^{NF}$ shown in Fig. 14A corresponds to a state in which the subjects do not face each other. The last period $T_{ij}^{F}$ corresponds to a state in which the subjects face each other. Fig. 14B shows a histogram of the synchronization rate $S_{ij}^{NF}$ obtained in the period $T_{ij}^{NF}$, and a histogram of the synchronization rate $S_{ij}^{F}$ obtained in the period $T_{ij}^{F}$.
When the i-th subject and j-th subject do not face each other, the second signals $S2_i$ and $S2_j$ vary at random. Accordingly, the synchronization rate $S_{ij}$ approaches zero. In this case, a histogram obtained from the multiple subject pairs has a peak at a position in the vicinity of zero. In contrast, when the subjects face each other, the second signals $S2_i$ and $S2_j$ show a marked tendency to vary in synchrony with each other. In this case, the synchronization rate $S_{ij}$ becomes a non-zero value, and accordingly, the histogram has a peak at a non-zero position. As can clearly be understood from Fig. 14B, it has been confirmed that there is a significant difference in the histogram of the synchronization rate $S_{ij}$ defined by Expression (1) between the state in which the subjects face each other and the state in which the subjects do not face each other. This means that the synchronization rate $S_{ij}$ can effectively be used as the third signal S3 that represents the interpersonal mental state.
With the embodiment 5 as described above, such an arrangement is capable of evaluating the interpersonal mental state directing attention to the frequency variation configured as a rhythm relationship value.
The evaluation apparatus 100 according to the embodiment measures communication between human beings so as to evaluate the quality of the communication. The evaluation results can be used to improve the quality of the communication. For example, the evaluation apparatus 100 is applicable to evaluation of the activity of communication between human beings. Also, with such an arrangement, an evaluation index may be calculated for evaluating group activity, and the evaluation index thus calculated may be used to improve the activity process or activity environment. Also, various kinds of applications of the present invention are conceivable, examples of which include: evaluation of educational effects obtained between a teacher and student; evaluation of a sense of understanding in a presentation; evaluation of a sense of trust in counseling; evaluation of empathy in consensus building; and the like. Also, the present invention is applicable to a watching service for preventing isolation or the like in a facility for the elderly.
Description has been made regarding the present invention with reference to the embodiment. The above-described embodiment has been described for exemplary purposes only, and is by no means intended to be interpreted restrictively. Rather, it can be readily conceived by those skilled in this art that various modifications may be made by making various combinations of the aforementioned components or processes, which are also encompassed in the technical scope of the present invention. Description will be made below regarding such modifications.

[Modification 1]

Detailed description has been made in the embodiments regarding an arrangement configured directing attention to nodding action as the non-verbal information that reflects the mental state of the subject. However, the present invention is not restricted to such an arrangement. Various kinds of non-verbal information that reflect the mental state may be employed as the first signal to be monitored. Specific examples of such non-verbal information include: visual information with respect to the subject that can be detected by means of an external device, such as back channeling in response, blinking, gaze retention time, body language, gestures, head shaking, trunk movement, and gaze direction movement; audio information with respect to the subject that can be detected by means of an external device, such as a turn-taking in a conversation, sighing, and tone of voice; non-verbal information with respect to speaking; and desired combinations of these. The kind of non-verbal information that reflects a given kind of mental state of a subject can be determined by those skilled in this art based on the results of study or an experimental rule obtained by experiment or inspection performed beforehand. Thus, it can be understood that suitable non-verbal information may preferably be selected according to the mental state to be evaluated.

[Modification 2]

Description has been made in the embodiments regarding an arrangement in which the amplitude of nodding action is extracted by calculating the time average of the amplitude of the first signal in order to evaluate the mental state. However, the present invention is not restricted to such an arrangement. Various modifications may be made for the rhythm analysis by means of the waveform analyzing unit 22. Conceivable specific examples of information used to evaluate an individual include the amplitude, frequency, kind of waveform, and frequency spectrum of the movement rhythm. Also, a multi-layered time scale may be employed. For example, a rhythm pattern represented by an envelope curve of a given rhythm may be employed as a higher-order rhythm pattern. Thus, the waveform analyzing unit 22 may generate the second signals S2a' and S2b' that reflect such information based on the first signals S1.

Also, the evaluation of a relationship between the mental states may be made giving consideration to the spatial position relationship between the multiple subjects 2, in addition to the synchrony relationship and the phase relationship between the rhythm patterns.

For example, let us consider a case in which multiple human beings have a conference or meeting. When they are having better communication, they tend to gather closer to each other. When a group member desires to make an assertion, he or she tends to sit down at a central position in the group. When a listener feels empathy for a speaker, the mutual distance tends to become closer. Conversely, when a listener feels antipathy for a speaker, the mutual distance tends to become wider. In business communication, in many cases, group members tend to have a conversation with a certain distance between them in a state in which they face each other. In contrast, in communication between familiar persons such as friends, they have a conversation in a state in which they are close to each other in the side-by-side direction. Thus, the spatial position relationship between the multiple subjects 2 may be employed as information that reflects the evaluation results of the mental state between the multiple subjects.

Also, the circadian rhythm (24-hour daily rhythm) may be used as the information that reflects the evaluation results of the relationship aspect and the individual aspect of the mental state. In social life, in many cases, there is synchrony between the daily activity rhythm patterns of individuals. In some cases, group members are partly compelled to synchronize with each other at a particular location such as an office or school. In some cases, group members voluntarily synchronize with each other at a particular location such as a house. Such a daily rhythm pattern may be evaluated in the same manner, thereby evaluating the mental state with respect to the relationship between the human beings. Such an activity rhythm is not restricted to a 24-hour daily rhythm. Also, the present invention is applicable to a weekly rhythm pattern, a monthly rhythm pattern, and an annual rhythm pattern.

[Modification 3]

Description has been made in the embodiments regarding an arrangement in which the relationship between the multiple subjects 2 is evaluated using signals obtained based on the same non-verbal information. However, the present invention is not restricted to such an arrangement. For example, first non-verbal information (e.g., nodding action) may be measured for one of multiple subjects, i.e., for the subject 2a, and second non-verbal information (e.g., gaze direction movement) may be measured for another one of multiple subjects, i.e., for the subject 2b. The relationship aspect of the mental state may be evaluated based on a relative relationship between the second signals S2a and S2b thus acquired using such respective methods.

[Modification 4]

As described in the embodiment 3, the multiple subjects may communicate with each other via a communication system. In recent years, in the fields of business and education, telecommunication between distant persons via a video phone system, voice telephone system, smartphone, tablet terminal, or the like, has become commonplace. The data obtained by the evaluation apparatus 100 can be used to evaluate such a voice telephone system and video phone system.

[Modification 5]

Description has been made in the embodiments regarding an arrangement in which communication between human beings is measured so as to evaluate their mental states. However, the present invention is not restricted to such an arrangement. Various kinds of applications are conceivable, examples of which include: design and evaluation of media that communicates with a user; evaluation of TV media programs; and the like. Examples of such media include TV programs, DVD software, electronic learning systems, and the like.

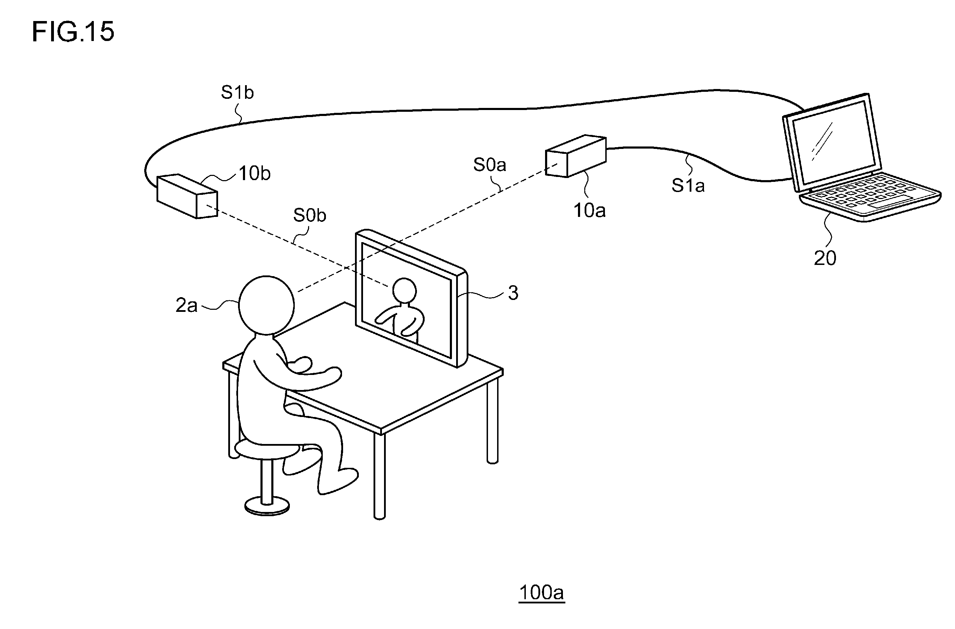

Fig. 15 is a diagram showing an evaluation apparatus 100a according to the modification 5.

The point of difference between the evaluation apparatus 100a shown in Fig. 15 and the evaluation apparatus 100 shown in Fig. 2 is that the subject 2b shown in Fig. 2 is replaced by a multimedia device 3 such as a computer, TV, tablet, or the like. With such an arrangement, a non-verbal information measurement unit 10b monitors the information dynamics, e.g., an audio signal or an image signal, provided to the subject 2a from the multimedia device 3, and generates a first signal Sib that corresponds to the information dynamics thus monitored.

Let us consider a case in which the evaluation apparatus 100a evaluates a learning software application to be employed in the field of education. In this case, the volume of an audio signal output from the multimedia device 3 may be used as information to be monitored. In this case, the evaluation apparatus 100a may measure the dynamics of the volume and non-verbal information that reflects the mental state of the subject 2a, and may evaluate the understanding level of the subject 2a based on the relative relationship between the measurement results.

As described above, such an evaluation apparatus may be employed as an evaluation system for evaluating various kinds of media including TV media. Also, such an evaluation result may be used as an index to develop a multimedia device such as a TV.

Description has been made regarding the present invention with reference to the embodiments using specific terms. However, the above-described embodiments show only the mechanisms and applications of the present invention for exemplary purposes only, and are by no means intended to be interpreted restrictively. Rather, various modifications and various changes in the layout can be made without departing from the spirit and scope of the present invention defined in appended claims.

[DESCRIPTION OF THE REFERENCE NUMERALS]

100 evaluation apparatus, 2 subject, 10 non-verbal information measurement unit, 20 signal processing unit, 22 waveform analyzing unit, 24 relationship evaluation unit, 26 individual evaluation unit.

[INDUSTRIAL APPLICABILITY]

The present invention is applicable to a technique for evaluating the mental state of a human being.

**Claims**

**1.** An evaluation apparatus that evaluates a relationship between a plurality of subjects in a communication between the subjects, the evaluation apparatus comprising:

a non-verbal information measurement unit that observes each of the plurality of subjects, and that generates first signals each of which is obtained as a time-series signal by quantifying non-verbal information obtained from the corresponding subject;
a waveform analyzing unit that generates, based on the first signals respectively obtained for the plurality of subjects, second signals each of which is configured as a value that relates to a feature configured as a rhythm of the non-verbal information with respect to the corresponding subject; and
a relationship evaluation unit that generates a third signal configured as an index that represents a mental state with respect to the relationship between the plurality of subjects, based on a relative relationship between the plurality of second signals that respectively correspond to the plurality of subjects.

2. The evaluation apparatus according to Claim 1, wherein the second signal is generated based on at least one from among frequency information and/or phase information with respect to the first signal.

3. The evaluation apparatus according to Claim 1 or 2, wherein the relative relationship between the plurality of second signals includes at least one of (i) degree of synchronization, (ii) phase difference, (iii) correlation relationship, (iv) frequency relationship, (v) phase relationship, (vi) amplitude relationship, and (vii) relationship between geometric features configured as waveform patterns.

4. The evaluation apparatus according to any one of Claims 1 through 3, wherein the relationship evaluation unit evaluates at least one from among empathy, sense of trust, sense of identification, sense of belonging, sense of reality, consensus or agreement, and sense of understanding.

5. The evaluation apparatus according to any one of Claims 1 through 4, wherein the relationship evaluation apparatus generates the third signal based on a spatial position relationship between the plurality of subjects, in addition to the relative relationship between the plurality of second signals.

6. The evaluation apparatus according to any one of Claims 1 through 5, further comprising an individual evaluation unit that generates a fourth signal configured as an index that represents a mental state for each of the plurality of subjects, based on the second signals respectively obtained for the plurality of subjects.

FIG.1A

FIG.1B

FIG.2

S1b
10b
10a
S1a
20
S0a
S0b
2a
2b

100

EP 2 932 899 A1

FIG.3

## FIG.4A

## FIG.4B

FIG.5A

FIG.5B

FIG.5C

@TS1

@TS2

r=0.007(n.s.)
WEAK CORRELATION

r=0.345
(P<0.001)
STRONG CORRELATION

FIG.6A

FIG.6B

## FIG.7A

## FIG.7B

FIG. 8

LOW LEVEL OF SYNCHRONY          HIGH LEVEL OF SYNCHRONY

SYNCHRONY
JUDGEMENT
RESULT
S3

1

0

120    240    360    480    600    720    840

TIME [sec]

LOW LEVEL OF AGREEMENT          HIGH LEVEL OF AGREEMENT

LEVEL OF
AGREEMENT

5

4

3

2

1

2    4    6    8    10    12    14

TIME [min]

EP 2 932 899 A1

FIG.9A

FIG.9B

FIG.9C

FIG.9D

FIG.10

FIG.11

FIG.12

FIG.13

FIG.14A

FIG.14B

FIG.15

EP 2 932 899 A1

100a

**INTERNATIONAL SEARCH REPORT**

| International application No. |
| --- |
| PCT/JP2013/007352 |

A. CLASSIFICATION OF SUBJECT MATTER
*A61B5/16*(2006.01)i

According to International Patent Classification (IPC) or to both national classification and IPC

B. FIELDS SEARCHED

Minimum documentation searched (classification system followed by classification symbols)
A61B5/16

Documentation searched other than minimum documentation to the extent that such documents are included in the fields searched
Jitsuyo Shinan Koho          1922–1996   Jitsuyo Shinan Toroku Koho   1996–2014
Kokai Jitsuyo Shinan Koho   1971–2014   Toroku Jitsuyo Shinan Koho   1994–2014

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
| --- | --- | --- |
| A | JP 2010-16796 A  (Sony Corp.),<br>21 January 2010 (21.01.2010),<br>entire text; all drawings<br>& US 2010/0079613 A1    & US 2009/0304289 A1<br>& CN 101600051 A | 1-6 |
| A | JP 2011-8393 A  (NEC Corp.),<br>13 January 2011 (13.01.2011),<br>entire text; all drawings<br>(Family: none) | 1-6 |
| A | JP 2012-79265 A  (NEC Corp.),<br>19 April 2012 (19.04.2012),<br>entire text; all drawings<br>(Family: none) | 1-6 |

| ☒ Further documents are listed in the continuation of Box C. | ☐ See patent family annex. |
| --- | --- |

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered    to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search<br>  29 January, 2014 (29.01.14) | Date of mailing of the international search report<br>  10 February, 2014 (10.02.14) |
| --- | --- |
| Name and mailing address of the ISA/<br>  Japanese Patent Office | Authorized officer |
| Facsimile No. | Telephone No. |

Form PCT/ISA/210 (second sheet) (July 2009)

**INTERNATIONAL SEARCH REPORT**

International application No.

PCT/JP2013/007352

C (Continuation). DOCUMENTS CONSIDERED TO BE RELEVANT

| Category* | Citation of document, with indication, where appropriate, of the relevant passages | Relevant to claim No. |
|---|---|---|
| A | JP 2012-128882 A (Hitachi, Ltd.), 05 July 2012 (05.07.2012), entire text; all drawings (Family: none) | 1-6 |

Form PCT/ISA/210 (continuation of second sheet) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2010022649 A
- JP 2007097668 A
- JP 2013052049 A